# EUROPEAN PATENT APPLICATION

(11) **EP 4 111 990 A1**
(43) Date of publication of application: **04.01.2023**
(21) Application number: 20939202.6
(22) Date of filing: 13.07.2020
(51) Int. Cl.: A61B 17/3211

(54) **CONNECTION MECHANISM FOR ULTRASONIC SCALPEL AND TRANSDUCER, AND ULTRASONIC SCALPEL ASSEMBLY EMPLOYING SAME**

(30) Priority: 04.06.2020 CN 202010502555
(71) Applicant: Surgnova Healthcare Technologies (Zhejiang) Co., Ltd., Cixi, Zhejiang 315300 (CN)
(72) Inventor: HUANG, Wenxing, Zhejiang 315300 (CN); ZHANG, Miao, Zhejiang 315300 (CN)
(74) Representative: Algemeen Octrooi- en Merkenbureau B.V.
(86) International application number: PCT/CN2020/101666
(87) International publication number: WO 2021/243809

(57) **Abstract**

The present disclosure provides a connection mechanism of an ultrasonic scalpel and a transducer and an ultrasonic scalpel assembly using the connection mechanism of an ultrasonic scalpel and a transducer. The connection mechanism of the present disclosure includes: a tightening turntable, a rotating cap and a sleeve structure. The sleeve structure has a hollow interior and is capable of accommodating the transducer and a cable. A surface of the sleeve structure is capable of forming a detachable sealing connection with a bag and/or a protective film, so that the sleeve structure is capable of being isolated from the outside by using the bag and/or the protective film, thus the transducer is protected from contamination. The design of the sleeve structure of the present disclosure may avoid the number of times of repeated sterilization of the transducer, shorten a damage to an instrument caused by the repeated sterilization of the instrument and facilitate an operation of an operator while meeting the requirements of a sterile surgery.

## Description

### TECHNICAL FIELD

The present disclosure relates to a field of medical instrument technology, and in particular to a connection mechanism of an ultrasonic scalpel and a transducer, and an ultrasonic scalpel assembly using the connection mechanism of an ultrasonic scalpel and a transducer.

### BACKGROUND

In an application technology of the ultrasonic scalpel, an ultrasonic scalpel head and the transducer are separate structures, and they are connected by a threaded joint. A tightening force is required to be controlled within a certain range during connecting, otherwise the ultrasonic scalpel may not work normally, or even the scalpel head or the transducer may be damaged. A commonly-used tightening method is to add a set of plastic torsion wrench. The torsion wrench consists of two components: a knob body and a rotating shaft. During use, a magnitude of the tightening force is controlled by using the knob body to press a plastic of the rotating shaft to deform elastically. During a surgery, it is required for a doctor to obliquely observe a selection of a magnitude gear from a side surface of the ultrasonic scalpel. During the surgery, a bag is prone to slip off when being directly sleeved on the ultrasonic scalpel, which may not safely and effectively isolate a contact between the doctor/user and a surface of the transducer, and an operation of the doctor's hand-held ultrasonic scalpel may be effected when the bag is directly tied to the ultrasonic scalpel. After the surgery, the transducer connected to the ultrasonic scalpel is required to be sterilized by high temperature.

In a process of implementing the present disclosure, the inventor found that the above-mentioned prior art has the following technical defects.
(1) each ultrasonic scalpel is provided with a torsion wrench, increasing a cost of an instrument;
(2) the torsion wrench is installed and removed through a front end of the instrument, which is inconvenient to operate;
(3) the torsion wrench is required to be kept properly during use, otherwise is easy to lose, which is very inconvenient in use;
(4) a torsion of the torsion wrench is provided by the elastic deformation of the plastic, a torsion value is unstable, thus the torsion value will become smaller and smaller after repeated use, and a plastic shrapnel is easy to break;
(5) since the torsion wrench is a separate accessory, the wrench may be forgotten to be sterilized during a disinfection process of the ultrasonic scalpel, so that the ultrasonic scalpel may not be connected for work according to set requirements and thus bring inconvenience;
(6) when the doctor is concentrating during surgery, it is required for the doctor to observe from the side surface to select the gear when operating buttons of the same shape.
(7) button positions include two gears of cutting and coagulating, the cutting is selected at most of the time during the doctor's actual operation, and a fatigue feel is stronger after long-term use due to a symmetrical position design.
(8) the bag directly protects the connection between the transducer and the ultrasonic scalpel, the fixing is unstable and is prone to slip off.
(9) the bag, when being directly installed on an outer surface of the ultrasonic scalpel, has an unstable sealing performance, which seriously affects the doctor's use of the ultrasonic scalpel.
(10) since a contact between the doctor/user and an outer surface of the transducer may contaminate the transducer during the surgery, it is necessary to repeat the high-temperature sterilization of the transducer after each surgery, however, the repeated high-temperature sterilization may seriously damage cables, components and performances of the transducer.

### SUMMARY

In view of this, a main objective of the present disclosure is to provide a connection mechanism of an ultrasonic scalpel and a transducer and an ultrasonic scalpel assembly using the connection mechanism of an ultrasonic scalpel and a transducer, so as to at least partially solve at least one of the above-mentioned technical problems.

In order to achieve the above-mentioned objectives, an aspect of the present disclosure provides a connection mechanism of an ultrasonic scalpel and a transducer, including:
a tightening turntable having a rotating body structure with an insertion direction of an ultrasonic scalpel as an axis, a center of the axis forming a through hole to sleeve the ultrasonic scalpel and enabling the ultrasonic scalpel to rotate synchronously with a rotating cap through a pin or a snap structure, wherein one end of the tightening turntable is provided with a thread or a snap structure for connecting with a sleeve structure accommodating the transducer; the other end of the tightening turntable is a limit plane provided with a plurality of grooves for matching with torsion pins installed on the rotating cap, or provided with a plurality of torsion pins for matching with grooves provided on the rotating cap, and the torsion pins are supported by springs and protruded from a surface of the tightening turntable;
the rotating cap having a rotating body structure with the insertion direction of the ultrasonic scalpel as an axis, a center of the axis center forming the through hole for the ultrasonic scalpel to pass through, wherein one side of the rotating cap facing the tightening turntable is provided with a plurality of torsion pins supported by springs and protruded from a surface of the rotating cap or a plurality of grooves correspondingly matched with the tightening turntable; the torsion pin on the tightening turntable or the rotating cap is capable of compressing the spring to be retracted to the surface of the tightening turntable or the rotating cap when the rotating cap is subjected to a rotational force, and is capable of extending into a corresponding groove when the rotating cap is not subjected to a force;
the sleeve structure having a hollow interior and capable of accommodating the transducer and a cable, wherein a surface of the sleeve structure is capable of forming a detachable sealing connection with a bag and/or a protective film, so that the sleeve structure is capable of being isolated from an outside by using the bag and/or the protective film, so that the transducer is protected from contamination.

Another aspect of the present disclosure further provides an ultrasonic scalpel assembly using the above-mentioned connection mechanism of an ultrasonic scalpel and an transducer.

Based on the above-mentioned solutions, the connection mechanism of the present disclosure has at least one of the following beneficial effects compared to the prior art.
(1) The tightening device is integrated with the ultrasonic scalpel, omitting the operation steps of installation and detachment and simplifying the operation.
(2) The tightening device is integrated with the ultrasonic scalpel, avoiding the risk of loss, a special storage is no longer required and simplifying the use.
(3) The tightening device is integrated with the ultrasonic scalpel, avoiding the risk of omission during disinfection.
(4) The torsion value is adjusted through the compression spring, so that the torque value is stable and reliable, and the service life is longer.
(5) The tightening device also acts as a rotating mechanism, and a separate torque wrench is no longer required, reducing a product cost.
(6) The buttons are special-shaped, which is helpful for an operator to distinguish between different gears when focusing on a surgery.
(7) The selection of the button positions takes into account ergonomics and an actual use of the doctor, which may be applied to more operations with different chirality and reduce a fatigue feel of the doctor.
(8) The sleeve design at a tail portion of the ultrasonic scalpel is convenient for an installation and a detachment for an instrument nurse, which may stably and effectively isolate a contact between the doctor and an outer surface of the transducer, while does not affect the doctor's use of the instrument.
(9) The sleeve design may reduce a harm of a long-term high-temperature sterilization to the cable of the transducer, prolong the service life of the transducer, and increase a single-day use time of a single transducer.

### BRIEF DESCRIPTION OF THE DRAWINGS

FIG. 1 is a schematic diagram of an overall structure of an ultrasonic scalpel assembly according to an embodiment of the present disclosure.
FIG. 2 is a schematic diagram of a semi-exploded structure of a connection mechanism according to an embodiment of the present disclosure.
FIG. 3 is a schematic diagram of a side semi-exploded structure of a connection mechanism according to an embodiment of the present disclosure.
FIG. 4A and FIG. 4B are schematic diagrams of a shape and a position of an ultrasonic scalpel button of a sleeve structure according to an embodiment of the present disclosure, respectively.
FIG. 5 is a schematic diagram of an overall structure of an ultrasonic scalpel assembly of a sleeve structure according to an embodiment of the present disclosure.
FIG. 6 is a schematic diagram of a semi-exploded structure of an ultrasonic scalpel assembly fusing a sleeve structure according to an embodiment of the present disclosure.

In the above-mentioned drawings, reference numerals have the following meanings:

| | | | |
|---|---|---|---|
| 01. | ultrasonic scalpel handle | 02. | tightening device |
| 03. | scalpel head assembly | 04. | tightening turntable |
| 05. | torsion pin | 06. | compression spring |
| 07. | rotating cap | 08. | decorative cover plate |
| 09. | ultrasonic scalpel button | 10. | sleeve structure |
| 11. | transducer | 12. | standard bag. |

### DETAILED DESCRIPTION OF EMBODIMENTS

In order to make the objectives, technical solutions and advantages of the present disclosure clearer, the present disclosure will be further described in detail below in conjunction with specific embodiments and with reference to the accompanying drawings.

The present disclosure provides a connection mechanism of an ultrasonic scalpel and a transducer, including:
a tightening turntable having a rotating body structure with an insertion direction of the ultrasonic scalpel as an axis, and a center of the axis forming a through hole to sleeve the ultrasonic scalpel and enabling the ultrasonic scalpel to rotate synchronously with a rotating cap through a pin or a snap structure. One end of the tightening turntable is provided with a thread or a snap structure for connecting with a sleeve structure accommodating the transducer; the other end of the tightening turntable is a limit plane provided with a plurality of grooves for matching with torsion pins installed on the rotating cap, or provided with a plurality of torsion pins for matching with grooves provided on the rotating cap, and the torsion pin is supported by a spring and protruded from a surface. The torsion wrench may be installed on any of the tightening turntable and the rotating cap, as long as the two may cooperate to limit a rotation;
the rotating cap having a rotating body structure with the insertion direction of the ultrasonic scalpel as an axis, and a center of the axis forming the through hole for the ultrasonic scalpel to pass through. One side of the rotating cap facing the tightening turntable is provided with a plurality of torsion pins or a plurality of grooves correspondingly matched with the tightening turntable. The torsion pin on the tightening turntable or the rotating cap may compress the spring to be retracted to the surface thereof when the rotating cap is subjected to a rotational force, and the torsion pin may extend into a corresponding groove when the rotating cap is not subjected to a force;
a sleeve structure having a hollow interior and capable of accommodating the transducer and a cable. A surface of the sleeve structure is capable of forming a detachable sealing connection with a bag and/or a protective film, so that the sleeve structure is capable of being isolated from the outside by using the bag and/or the protective film, so that the transducer is protected from contamination.

The design of the sleeve structure may isolate a sterilized transducer from a user and a patient without a direct contact. During operation, a bag is fixed on a rear edge protrusion of the sleeve structure in a sterile state, the transducer passes through the bag and a sleeve, and is connected to the ultrasonic scalpel normally. After use, the transducer is removed first, and then the sleeve is unscrewed. After the sleeve and bag are replaced, the next operation may be performed without a further high-temperature sterilization.

In a preferred embodiment, the sleeve structure is a cylindrical hollow cylinder, the rear edge protrusion of the sleeve structure forms a ring of expanded flange structure, a detachable sealing connection with the bag and/or the protective film may be formed on the expanded flange structure, so that an interior of the sleeve structure may be isolated from the outside by using the bag and/or the protective film. The sleeve structure is provided with a snap, and the bag is rotated about 30° to be fixed on a middle transducer sleeve in a sterile state. The transducer sequentially passes through the bag and the sleeve so as to be connected to the ultrasonic scalpel normally. After use, the transducer is removed, and then the sleeve is reversely rotated and unscrewed to exit the connection with the ultrasonic scalpel. Since the sleeve structure completely isolates an outer surface of the transducer from the doctor, a contamination of the outer surface of the transducer may be effectively avoided while an operation and use of the doctor's instrument remain unaffected, thus a harm of a long-term high-temperature sterilization to the cable of the transducer may be reduced, and a service life of the transducer may be prolonged.

A plurality of protruding ribs may be formed on a side surface of the tightening turntable and/or the rotating cap so as to facilitate holding and exerting a force. A material of the tightening turntable and/or the rotating cap may be an engineering plastic or metal, such as polyethylene PE, polypropylene PP, ABS, PET, aluminium alloy, etc.

The torsion pin supported by the spring and protruded from the surface may be a hemispherical or spherical bead disposed on a cube connected through a straight spring, or may directly use a ready-made spring snap bead. The number of the torsion pins may be 1, 2, 3, 4, 5, 6, 7 ..., preferably 2 or 3. A control of a plurality of different angles may be realized by providing a cooperation of the plurality of torque pins and the grooves. For example, the torsion pin is slid into different grooves for snapping.

Preferably, a head portion of the torsion pin is provided to form a smooth transition at an edge in a clockwise direction of an outer circumference tangent and form a step at an edge in a counterclockwise direction, and the groove is provided similarly. In this way, when the tightening turntable and/or the rotating cap is rotated clockwise, the ultrasonic scalpel is driven to rotate by a pin shaft of the tightening turntable, so that the ultrasonic scalpel is threaded with the sleeve structure. A force is further applied after the thread is fully screwed in, and the torsion pin is subjected to an increasing tangential force and an increasing positive pressure. When a compression spring is compressed, a torsion value increases continuously with an increase of a compression amount of the compression spring; when a certain amount of compression is reached, the head portion of the torsion pin is separated from the groove on the tightening turntable, and a limiting effect disappears, thus the torsion pin plays a role of torsion overload protection. When detaching the transducer, the rotating cap is rotated counterclockwise, and the tightening turntable is driven to rotate together by a top plane of the torsion pin, so that the thread is loosened.

Preferably, the connection mechanism of the ultrasonic scalpel and the transducer may further includes a pistol-shaped handle which is convenient for an operator to hold, especially for a single-handed operation. The rotating cap and the tightening turntable are located at a muzzle part of the pistol-shaped handle, the sleeve structure is located at a barrel part, and at least two ultrasonic scalpel buttons are disposed at a trigger part of the pistol-shaped handle, and the at least two ultrasonic scalpel buttons are provided in different shapes, sizes and pressing angles according to frequencies of use and habits so as to be used for different gear selections. Further preferably, the number of the ultrasonic scalpel buttons is two, and action stroke of the ultrasonic scalpel buttons are provided by an eccentricity deviating from a horizontal line of a button center. In this way, a special-shaped design of the button is helpful for the operator to distinguish between different gears when focusing on the surgery; and a special-shaped position of the button may be applied to operations with different chirality and reduce a fatigue feel of the doctor.

In addition, a decorative cover plate for decoration is further sleeved on the rotating cap to enhance a beauty of the connection mechanism and play a role of waterproofing and dustproofing.

The present disclosure further provides an ultrasonic scalpel assembly using the above-mentioned connection mechanism, which includes a transducer, an ultrasonic scalpel, etc.

The technical solution of the present disclosure will be further described below through specific embodiments in conjunction with the accompanying drawings. It should be noted that the following embodiments are only for illustration, and are not intended to limit the present disclosure.

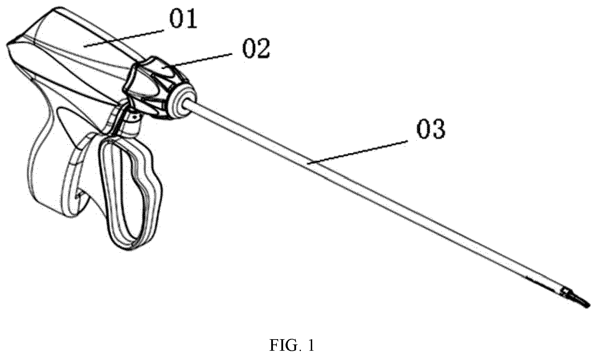

FIG. 1 is a schematic diagram of an overall structure of an ultrasonic scalpel of a connection mechanism according to an embodiment of the present disclosure. As shown in FIG. 1, 01 is an ultrasonic scalpel handle (with a transducer inside), 02 is a tightening device, which includes the above-mentioned tightening turntable and the rotating cap, and 03 is a scalpel head assembly mainly including the ultrasonic scalpel. The tightening device 02 is fixed on the ultrasonic scalpel handle 01 through a snap and is fixed to 03 through a pin shaft, thus the tightening device 02 is rotated by toggling, and 03 is driven to rotate at the same time.

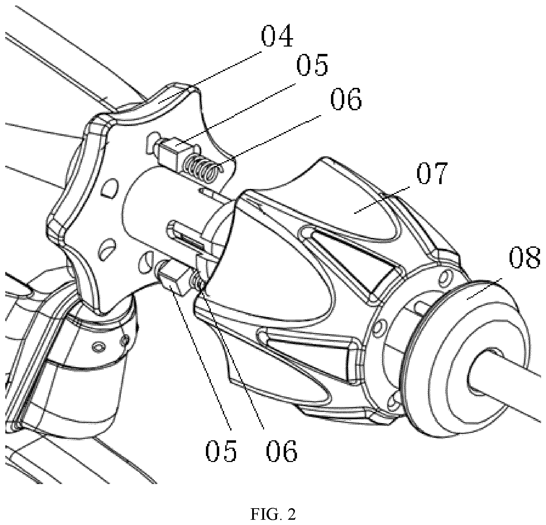

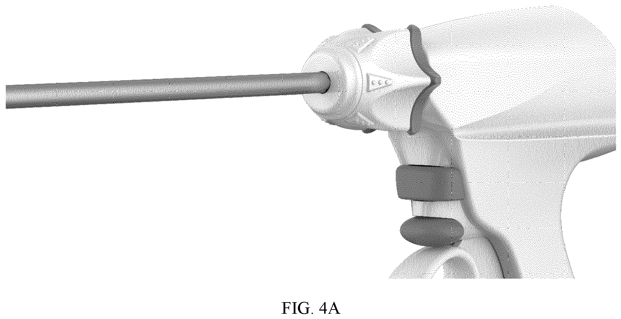

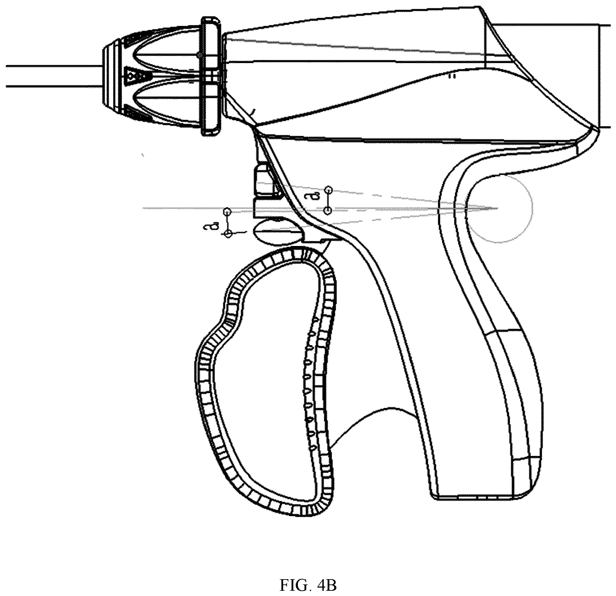

FIG. 2 is a schematic diagram of a semi-exploded structure of a connection mechanism according to an embodiment of the present disclosure. As shown in FIG. 2, 04 is a tightening turntable, which is an approximate hexagon with six rounded corners, and six semicircular grooves are provided on a side of the tightening turntable facing a rotating cap; 05 is a torsion pin, a head portion of the torsion pin is semicircular so as to be used in conjunction with the groove on the tightening turntable 04; 06 is a compression spring; 07 is the rotating cap, which may be rotated independently, and the torsion pin 05 and the compression spring 06 are assembled in corresponding positions in the rotating cap 07; and 08 is a decorative cover plate which plays a role of decorating and supporting a scalpel bar.

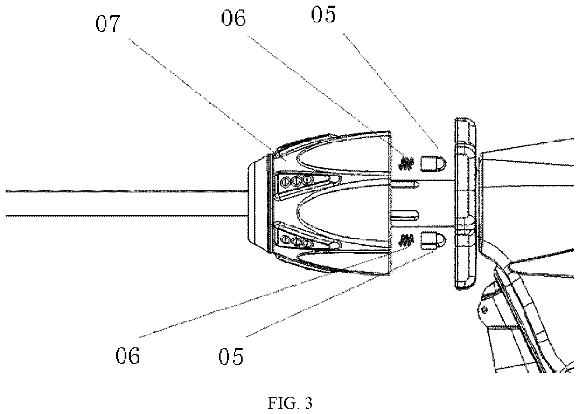

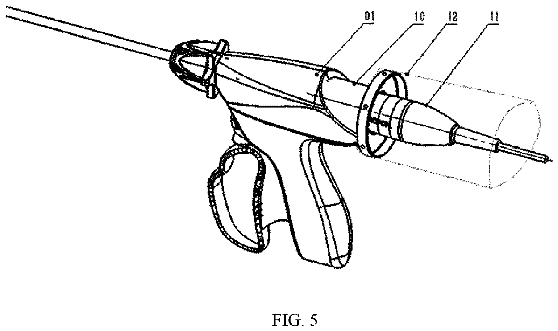

FIG. 3 is a schematic diagram of a side semi-exploded structure of a connection mechanism according to an embodiment of the present disclosure. As shown in FIG. 3, 05 is a torsion pin, a head portion of the torsion pin is semicircular so as to be used in conjunction with a groove on a tightening turntable 04, 06 is a compression spring, 07 is a rotating cap, and the torsion pin 05 and the compression spring 06 are assembled in corresponding positions in the rotating cap 07.

FIG. 4A and FIG. 4B are schematic diagrams of a shape and a position of an ultrasonic scalpel button of an ultrasonic scalpel handle portion according to an embodiment of the present disclosure. As shown in FIG. 4A, the number of the ultrasonic scalpel buttons 09 is two, outer contours of the two ultrasonic scalpel buttons are not the same and touch feelings of the two ultrasonic scalpel buttons are obviously different. As shown in FIG. 4B, a center line of button gears of the two ultrasonic scalpel buttons 09 are also provided downward, that is, a previous button is a commonly-used button, which occupies a larger operation area and is easier to touch. Operation strokes of the two ultrasonic scalpel buttons are not provided horizontally, but are properly eccentric. For example, an included angle of the operation stroke relative to a horizontal line is a, which is easier for the operator to distinguish between the two ultrasonic scalpel buttons when operating with a single hand.

FIG. 5 is a schematic diagram of an overall structure of an ultrasonic scalpel assembly fusing a sleeve structure according to an embodiment of the present disclosure. 10 is a sleeve structure, 11 is a transducer, and 12 is a standard bag. Before a surgery, a sterile ultrasonic scalpel, the sleeve structure 10 and the standard bag 12 are disassembled, the standard bag 11 is fixed on the sleeve structure 10, and the sleeve structure 10 is rotatably fixed to the ultrasonic scalpel handle 01. After that, the sterilized transducer 11 sequentially passes through the standard bag 12 and the sleeve structure 10 so as to be connected to the ultrasonic scalpel. After the surgery is completed, the transducer 11 is first unscrewed to exit, and then the sleeve structure 10 is unscrewed. The sleeve structure 10 and standard bag 12 are treated in accordance with a medical waste standard. The sterilized transducer 11 may be protected from contamination, and the next operation may be continued, saving a sterilization time, and prolonging a service life of a cable of the transducer 11.

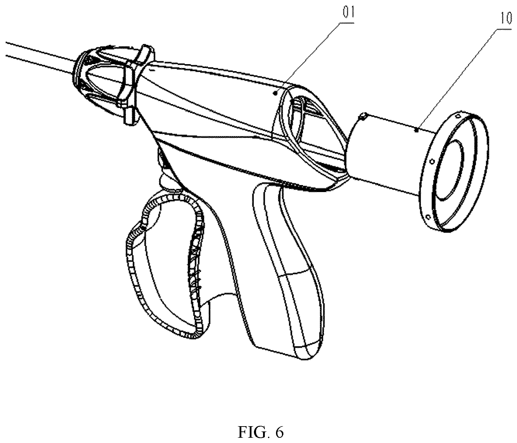

FIG. 6 is a schematic diagram of a semi-exploded structure of an ultrasonic scalpel assembly fusing a sleeve structure according to an embodiment of the present disclosure. 10 is a sleeve structure, and 01 is an ultrasonic scalpel handle; there are two convex wedge-shaped designs on the sleeve structure 10, thus a clockwise fixation is more secure and a counterclockwise detachment is easier.

Working principles of the connection mechanism of the ultrasonic scalpel and the transducer and the ultrasonic scalpel using the connection mechanism of the ultrasonic scalpel and the transducer of the present disclosure are as follows:
1. When assembling the ultrasonic scalpel and the transducer, the rotating cap 07 is rotated clockwise. Since the torsion pin 05 has a limiting effect under an action of a tangential force, the tightening turntable 04 is driven to rotate at the same time, and the tightening turntable 04 drives the scalpel head assembly 03 to rotate through the pin shaft, so that the ultrasonic scalpel is threaded with the transducer. A force is further applied after the thread is fully screwed in, the torsion pin 05 is subjected to an increasing tangential force and an increasing positive pressure, the torsion pin 05 compresses the compression spring 06 downward, the torsion value increases continuously with an increase of the compression amount of the compression spring 06. When a certain amount of compression is reached, the head portion of the torsion pin 05 is separated from the groove on the tightening turntable 04, and the limiting effect disappears, thus the torsion pin plays a role of torsional overload protection. When detaching the transducer, the rotating cap 07 is rotated counterclockwise, and the tightening turntable 04 is driven to rotate together by a top plane of the torsion pin 05, so that the thread is loosened.
2. During the use of the ultrasonic scalpel, the doctor may adjust the angle of the scalpel head by rotating the tightening turntable 04 or the rotating cap 07.
3. A mechanism design of the sleeve structure 10 on the ultrasonic scalpel handle isolates the sterilized transducer 11 from the user and the patient without a direct contact. In a sterile state, the standard bag 12 is fixed on the sleeve structure 10, the sleeve structure 10 is rotatably fixed on a tail portion of the ultrasonic scalpel handle, and the transducer 11 sequentially passes through the standard bag 12 and the sleeve structure 10 so as to be connected to the ultrasonic scalpel normally. After use, the transducer 11 is removed first, and then the sleeve structure 10 is unscrewed in an opposite direction. The next operation may be performed after replacing the sterile sleeve structure 10 and the sterile standard bag 12.

In addition, the above-mentioned definitions of various elements and methods are not limited to the various specific structures, shapes or methods mentioned in the embodiments, and those skilled in the art may make simply modifications or replacements. For example: the decorative cover plate may be omitted; the number of the pins and the compression springs may be flexibly provided according to design requirements; the tightening device may be replaced by other structures; the special-shaped buttons may be replaced by other structures; and the structure of the sleeve may be flexibly designed and replaced according to appearance requirements of the ultrasonic scalpel.

After verification by experiments, the design of the sleeve structure of the present disclosure may avoid the number of times of repeated sterilization of the transducer and shorten a damage to an instrument caused by the repeated sterilization of the instrument while meeting requirements of a sterile surgery. In addition, the sleeve design may be easily installed and detached to facilitate an operation of an instrument nurse. After installation, a doctor's hand feeling and operating comfort of the instrument may not be affected. Besides, the connection mechanism of the present disclosure is integrated with the ultrasonic scalpel, omitting operation steps of installation, detachment and disinfection, thus eliminating a risk of loss, the torsion value is stable and reliable, thus a service life may be longer. The button design of the present disclosure conforms to ergonomics and reduces a burden of the operator.

The above-mentioned specific embodiments have further described in detail the objectives, technical solutions and beneficial effects of the present disclosure. It should be understood that the above are only specific embodiments of the present disclosure, and they are not intended to limit the present disclosure. Any modifications, equivalent substitutions, improvements, and the like made within the spirit and scope of the present disclosure shall be included in the scope of protection of the present disclosure.

## Claims

1. A connection mechanism of an ultrasonic scalpel and a transducer, comprising:
a tightening turntable having a rotating body structure with an insertion direction of an ultrasonic scalpel as an axis, a center of the axis forming a through hole to sleeve the ultrasonic scalpel and enabling the ultrasonic scalpel to rotate synchronously with a rotating cap through a pin or a snap structure, wherein one end of the tightening turntable is provided with a thread or a snap structure for connecting with a sleeve structure accommodating the transducer; the other end of the tightening turntable is a limit plane provided with a plurality of grooves for matching with torsion pins installed on the rotating cap, or provided with a plurality of torsion pins for matching with grooves provided on the rotating cap, and the torsion pins are supported by springs and protruded from a surface of the tightening turntable;
the rotating cap having a rotating body structure with the insertion direction of the ultrasonic scalpel as an axis, a center of the axis center forming the through hole for the ultrasonic scalpel to pass through, wherein one side of the rotating cap facing the tightening turntable is provided with a plurality of torsion pins supported by springs and protruded from a surface of the rotating cap or a plurality of grooves correspondingly matched with the tightening turntable; the torsion pin on the tightening turntable or the rotating cap is capable of compressing the spring to be retracted to the surface of the tightening turntable or the rotating cap when the rotating cap is subjected to a rotational force, and is capable of extending into a corresponding groove when the rotating cap is not subjected to a force;
the sleeve structure having a hollow interior and capable of accommodating the transducer and a cable, wherein a surface of the sleeve structure is capable of forming a detachable sealing connection with a bag and/or a protective film, so that the sleeve structure is capable of being isolated from an outside by using the bag and/or the protective film, so that the transducer is protected from contamination.

2. The connection mechanism of an ultrasonic scalpel and a transducer according to claim 1, wherein a plurality of protruding ribs are formed on a side surface of the tightening turntable and/or the rotating cap so as to facilitate holding and exerting a force.

3. The connection mechanism of an ultrasonic scalpel and a transducer according to claim 1, wherein a material of the tightening turntable and/or the rotating cap is an engineering plastic or metal, preferably polyethylene PE, polypropylene PP, ABS, POM or aluminium alloy.

4. The connection mechanism of an ultrasonic scalpel and a transducer according to claim 1, wherein the torsion pin supported by the spring and protruded from the surface are hemispherical or spherical beads disposed on a cube connected through a straight spring, or directly use a spring snap bead.

5. The connection mechanism of an ultrasonic scalpel and a transducer according to claim 1, wherein the number of the torsion pins is 1, 2, 3, 4, 5, 6 or 7.

6. The connection mechanism of an ultrasonic scalpel and a transducer according to claim 1, wherein a head portion of the torsion pin is provided to form a smooth transition at an edge in a clockwise direction of an outer tangent and form a step at an edge in a counterclockwise direction, and the groove is provided in the same way, so that when the tightening turntable and/or the rotating cap is rotated clockwise, the ultrasonic scalpel is driven to rotate by a pin shaft of the tightening turntable, so that the ultrasonic scalpel is threaded with the sleeve structure; a force is further applied after the thread is fully screwed in, and the torsion pin is subjected to an increasing tangential force and an increasing positive pressure; when a compression spring is compressed, a torsion value increases continuously with an increase of a compression amount of the compression spring; when a certain amount of compression is reached, the head portion of the torsion pin is separated from the groove on the tightening turntable, and a limiting effect disappears, thus the torsion pin plays a role of torque overload protection; when detaching the transducer, the rotating cap is rotated counterclockwise, and the tightening turntable is driven to rotate together by a top plane of the torsion pin, so that the thread is loosened.

7. The connection mechanism of an ultrasonic scalpel and a transducer according to claim 1, further comprising a pistol-shaped handle, wherein the rotating cap and the tightening turntable are located at a muzzle part of the pistol-shaped handle, the sleeve structure is located at a barrel part of the pistol-shaped handle, and at least two ultrasonic scalpel buttons are disposed at a trigger part of the pistol-shaped handle, wherein the ultrasonic scalpel buttons are provided in different shapes, sizes and pressing angles according to frequencies of use and habits so as to be used for different gear selections.

8. The connection mechanism of an ultrasonic scalpel and a transducer according to claim 7, wherein the number of the ultrasonic scalpel buttons is two, and action strokes of the ultrasonic scalpel buttons are provided by an eccentricity deviating from a horizontal line of a button center.

9. The connection mechanism of an ultrasonic scalpel and a transducer according to claim 1, wherein a decorative cover plate for decoration is further sleeved on the rotating cap to enhance a beauty of the connection mechanism and play a role of waterproofing and dustproofing.

10. The connection mechanism of an ultrasonic scalpel and a transducer according to claim 1, wherein the sleeve structure forms a flange structure at an end portion opposite to the tightening turntable for sealing and fixing the bag.

11. An ultrasonic scalpel assembly using the connection mechanism of an ultrasonic scalpel and a transducer according to any one of claims 1 to 10.
